(19) **Europäisches Patentamt**
**European Patent Office**
**Office européen des brevets**

(11) **EP 2 113 282 B1**

(12) **EUROPEAN PATENT SPECIFICATION**

(45) Date of publication and mention
of the grant of the patent:
**28.11.2012 Bulletin 2012/48**

(51) Int Cl.:
*A61M 35/00* (2006.01)     *A61K 8/02* (2006.01)
*A45D 44/00* (2006.01)

(21) Application number: **09251008.0**

(22) Date of filing: **31.03.2009**

(54) **Facial mask**

Gesichtsmaske

Masque facial

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **31.03.2008 US 59273**

(43) Date of publication of application:
**04.11.2009 Bulletin 2009/45**

(73) Proprietor: **Johnson & Johnson Consumer Companies, Inc.**
**Skillman, NJ 08558 (US)**

(72) Inventors:
• **Beatty, Heidi**
**Hillsborough, NJ 08844 (US)**

• **Formosa, Dan**
**Pirmont, NY 10968 (US)**
• **Francoeur, Julie**
**Quebec, CA H1V 2N8 (CA)**
• **Uozumi, Sachiko**
**New York, NY 10011 (US)**

(74) Representative: **Kirsch, Susan Edith et al**
**Carpmaels & Ransford**
**One Southampton Row**
**London**
**WC1B 5HA (GB)**

(56) References cited:
WO-A-01/01952          FR-A- 2 870 698
JP-A- 2000 287 751      US-A- 5 026 552
US-A1- 2005 074 484    US-A1- 2008 147 022

**Description**

**FIELD OF THE INVENTION**

The present invention relates to a facial mask and uses thereof to treat skin.

**BACKGROUND OF THE INVENTION**

Products such as cleansers and moisturizers formulated with vitamins and other skin benefit agents have been used for many years to treat the skin. Employing a water-insoluble substrate such as wipe or mask to assist in the process of cleansing, moisturizing and delivery of certain benefit agents to the skin is also known. For example, consumers typically use hydrating facial mask products for treatment of various skin conditions as well as to improve the physical appearance and texture of the facial skin. This can be accomplished while the user relaxes, such as in a prone position, while the mask contacts the skin of the face, and provides benefits thereto.

The dimensions of the human face vary considerably from individual to individual. Conventional facial mask products can be grossly inadequate in their ability to fit a wide variety of facial shapes and sizes, yet still lie flatly against the face. In particular, conventional approaches to accommodate variance in facial size, i.e., the inclusion of conventional slits, does little to meet this pressing need.

For example, a conventional facial mask spread out fully on small face, will touch the hairline. This is problematic especially for typical users who use the mask immediately before sleeping and do not want their hair wet with a skin treatment composition. If the facial mask is applied so as not to wet the hairline it must be "bunched up," thereby leaving bubbles between the mask and the skin. This situation could give rise to uneven treatment of the face with the skin care composition. Such uneven treatment is especially problematic for facial masks that are designed to lighten or even the tone of the skin. For users with larger faces, the situation is also problematic since conventional facial masks are not be able to cover the entire face - also leaving portions of the skin untreated. Furthermore, conventional facial masks tend to hang on the face and provide little gripping and firming, and are prone to fall off during use.

Applicants have now developed a facial mask that is adaptable in size and shape for a variety of faces and also adheres well to the face. The facial mask comprises a water-insoluble substrate having one or more separation features, such as slits, adapted to allow portions of the water-insoluble substrate to overlap and adhere to one another. In one embodiment, the water-insoluble substrate comprises at least one separation feature that forms a laterally-extending tab having a hinge area of at least 0.5 cm$^2$. In another embodiment, the separation feature defines an angle of disposition greater than 0°. The separation features enable the creation of substantial hinges in the facial mask that function to increase its adaptability for a wide variety of faces, resulting in greater comfort, better overall adherence to the skin, and a pleasant lifting/firming sensation. Furthermore, in certain embodiments, one or more of these advantages can be achieved with the convenience of a single-piece mask.

It is known in the art to provide small slits in facial masks. For instance US20060104931A1, US20050013784A1, US20040018166A1, EP1357819B1, WO 01/01952 A1 and FR 2 870 698 A1 disclose such facial masks. Several commercially available facial masks comprising slits also exist. NEUTROGENA Deep Hydrating Mask comprises a slit between the eye and mouth regions. It comprises a 3.5 cm slit on the side at the chin at an angle of less than about 5°. The PEARL SILK W-Cut, Oil Control & White Clear Mask facial mask is a two-piece mask with a 2.5 cm slit and a 1 cm kink in the chin portion. The kink bends backwards, away from the centerline of the mask.

None of these facial masks, however, comprise slits or other features adapted to form tabs and hinges capable of overlapping other parts of the masks. As such, their utility for different facial shapes is limited.

**BRIEF DESCRIPTION OF THE DRAWINGS**

**FIG.1** is a top plan view of a facial mask in accordance with a first embodiment of the present invention;

**FIG. 2** is a top plan view of the facial mask of **FIG. 1,** showing additional features thereof;

**FIG. 3** is side perspective view of a user wearing the facial mask of **FIG. 1**;

**FIG. 4** is a top plan view of a facial mask in accordance with a second embodiment of the present invention;

**FIG. 5** is side perspective view of a user wearing the facial mask of **FIG. 4;**

**FIG. 6** is a top plan view of a facial mask in accordance with a third embodiment of the present invention,;

**FIG. 7** is a top plan view of a facial mask in accordance with a fourth embodiment of the present invention;

**FIG. 8** is side perspective view of a user wearing the facial mask of **FIG. 7;** and

**FIG. 9** is a top plan view of a two-part facial mask in accordance with a fifth embodiment of the present invention.

## SUMMARY OF THE INVENTION

In one aspect of the invention, a facial mask is provided comprising a unitary, water-insoluble substrate sized and shaped to lie against and substantially coincident with an entire face of a human user, said water-insoluble substrate defined by a perimeter and comprising a forehead region, a mid region, and a chin region, wherein said water-insoluble substrate further comprises at least one separation feature that forms a laterally-extending tab having a hinge area of at least 0.5 cm$^2$.

The present invention also provides a facial mask comprising a water-insoluble substrate sized and shaped to lie against and substantially coincident with a face of a human user, said water-insoluble substrate defined by a perimeter and comprising a forehead region, a mid region, and a chin region, wherein said water-soluble substrate comprises at least one separation feature extending from said perimeter into either said forehead region or said chin region, and wherein said separation feature defines an angle of disposition greater than 0°.

The present invention further provides a method of treating the facial skin of a human user, comprising placing a water-insoluble substrate against the facial skin of said user such that said water-insoluble substrate covers substantially all of said facial skin, wherein a first portion of said water-insoluble substrate overlaps a second portion of said water-insoluble substrate, whereby said first portion and said second portion adhere to one another. The method is non-therapeutic.

## DETAILED DESCRIPTION OF THE INVENTION

It is believed that one of ordinary skill in the art can, based upon the description herein, utilize the present invention to its fullest extent. The following specific embodiments of the invention are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Whenever used, any percentage is weight by weight (%w/w) unless otherwise indicated.

Water-Insoluble Substrate

The facial mask comprises a water-insoluble substrate. As used herein, "water-insoluble" means the substrate, upon immersion in distilled water at 25° C, does not readily dissolve or readily break apart. While portions of the water-insoluble substrate may be leachable or readily soluble in the distilled water, at least one portion of the water-insoluble substrate remains intact. For example, the intact portion may be readily manipulated, such as picked up and transported as an interconnected cohesive unit, by a user.

A wide variety of materials can be used as the water-insoluble substrate. Examples of suitable substrates include, but are not limited to, fibrous and/ or porous substrates such as substrates including or formed from non-woven fibers, woven fibers, hydro-entangled fibers, or air-entangled fibers. The water-insoluble substrate may include natural sponges, synthetic sponges, and polymeric netted meshes.

The water-insoluble substrate is preferably formed to retain a liquid impregnate (such as by absorbing the liquid impregnate among, along, and/or between fibers comprising the water-insoluble substrate) for a period of time at least as long as from when the product is manufactured to a time when the product is used by a consumer (*i.e.,* a shelf storage period). In this embodiment of the invention, during this shelf storage period the water-insoluble substrate should generally maintain its mechanical integrity such that a user can lay the water-insoluble substrate onto the skin and transfer liquid impregnate thereto. Furthermore the water-insoluble substrate is generally capable of holding portions of the liquid impregnate against the facial skin when used.

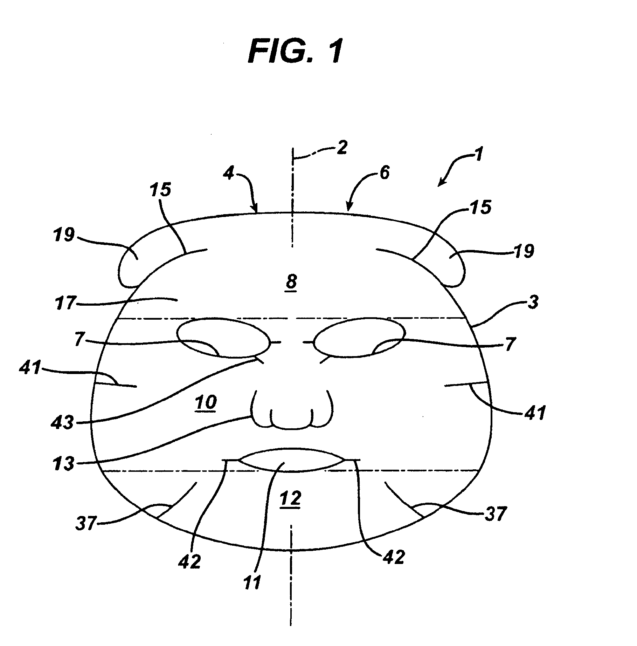

**FIG. 1** depicts one embodiment of a facial mask consistent with embodiments of the invention described herein. Facial mask 1 is generally sized and shaped to lie against the face of a user. It is preferred that the facial mask 1 lie substantially flatly against the face of the user, i.e., the facial mask 1 is capable of draping across the face and generally conforming to the curvature of the face. The facial mask is generally further capable of lying substantially coincident with the face of the user, i.e., it generally only requires simple manipulation such as unfolding or at most slight tearing of preformed perforations in order to assume a shape that coincides with a human face. In a preferred embodiment, the facial mask is sized and shaped to lie substantially flatly against the entire face of a user. As used herein, "entire face" means the majority, e.g., at least about 90%, of the face, including the nose, cheeks, around the eyes, and the forehead, as well as under and above the mouth.

The facial mask 1 may further comprise a vertical centerline, preferably a vertical axis of symmetry 2 that separates a left side 4 from a right side 6 of the facial mask 1. Furthermore, the facial mask 1 comprises a perimeter 3 that defines the outer boundary of the facial mask when it is laid flat such as on a tabletop.

The facial mask 1 may include, within the perimeter 3, at least one opening. The opening may be pre-cut, in which case it will be visible as area devoid of water-insoluble substrate. Alternatively, the opening may be defined by a perforated or weakened line. In this embodiment, the use can readily separate, that, using little force and in a controlled fashion, the water-insoluble substrate along the perforated or weakened line. For example, a user may "punch" or "tear" the water-insoluble substrate along such a line or other shape in a controlled fashion prior to use.

As shown in **FIG.** 1, the facial mask 1 includes pre-cut openings 7 for the eyes of a user. It is further desirable that facial mask 1 include a pre-cut opening I 1 for the mouth of the user.

The facial mask may further include at least one interior slit. As used herein, an "interior slit" is a pre-cut line in the water-insoluble material within but not touching the perimeter. Interior slits are formed by slicing the water-insoluble substrate when laid flat and are often visible only as lines or boundaries when the facial mask is laid flat. For example, facial mask 1 includes one or more interior slits 13 that permit the nostril area or the mouth of the user to be exposed (for instance, uncovered) in use.

Interior slits 13 may be straight or curved. In one embodiment, in order to provide better fit, one or more interior slits 13 for the nose may include a plurality of arcuate portions to facilitate adherence to an underside of the user's nasal ridge, rather than, in the case of a user with a small nose, dangling or hanging off of the nose.

The facial mask 1 includes a forehead region 8 comprising that portion of the facial mask 1 entirely above the openings 7 for the eyes; a chin region 12 comprising that portion entirely below the opening 11 for the mouth; and a mid region 10 that includes all other portions of the facial mask 1.

Note that the perimeter 3 is generally of a gently curving, primarily arcuate shape that generally outlines or conforms to the shape of a typical human face. In accordance with the invention, the perimeter 3 may also encompass various features in order to enhance the fit and comfort for users having a wide range of facial shapes and sizes.

Specifically, the facial mask comprises at least one separation feature. As used herein, a "separation feature" is a feature defined by a perforated or weakened line that can be readily separated by the user, or a pre-cut line, in the water-insoluble substrate that touches the perimeter. The separation feature may, for example, be a slit, notch or wedge, or other indentation or protrusion along the perimeter having a regular or irregular shape.

For example, the facial mask may include one or more slits touching the perimeter as separation features. As shown in **FIG. 1**, facial mask 1 includes a pair of slits 15 that are symmetric about, i.e., "reflected about" the axis of symmetry 2. Slits 15 are such that they form laterally-extending tabs 19 in the forehead region 8.

**FIG**. 2 is another plan view of the facial mask 1 of **FIG. 1** showing additional features thereof. Slit 15 is defined by an initiation point A where the slit intersects perimeter 3, and a vertex V where the slit 15 terminates. Slit 15 generally has a component that is tangent to the perimeter 3 and directed towards the axis of symmetry 2. The degree of the tangency of slit 15 can be determined by the magnitude of its angle of disposition 23. The angle of disposition 23 is determined by locating the geometric centroid 31 of the facial mask 1. The geometric centroid 31 is the "center of area" point of the mask, as it lays flat on a table top. Although the geometric centroid may vary depending upon the design of the facial mask, it is often near the nose slits. Geometric centroid, $r_{x,y}$ can be calculated by integrating over the area having spatial coordinates x,y of the mask:

$$r_{x,y} = 1/\text{Area} \left( \int_A r_{x,y} \, d\text{Area} \right)$$

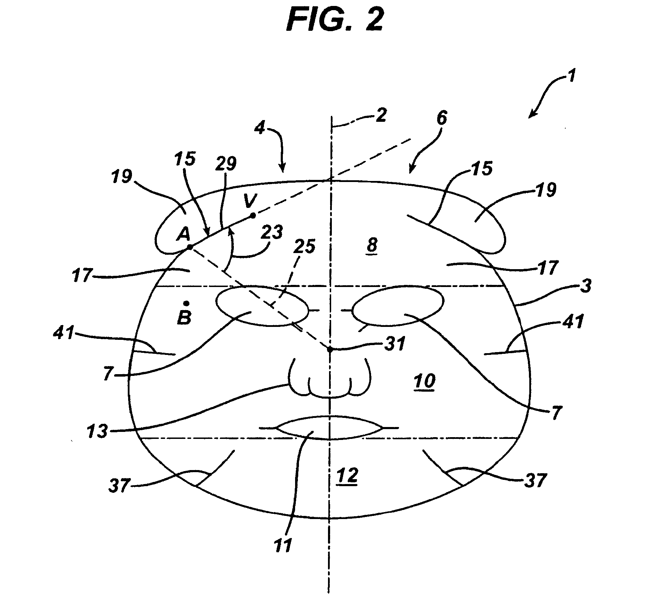

In **FIG. 2** ray 25 connects the geometric centroid 31 and the initiation point A of the slit 15. A second ray 29 connects initiation point A and vertex V. If the slit is curved, ray 29 is constructed tangent to slit 15. Angle of disposition 23 at any point on slit 15 is determined as the angle between ray 25 and ray 29. The sense (sign) of the angle of disposition 23 is positive if, in effecting a rotation from ray 25 to ray 29 about initiation point A, one rotates towards the axis of symmetry 2.

Referring again to **FIG. 2,** angle of disposition 23 is positive. In contrast, if slit 15 instead connected points A and B, the angle of disposition would be negative. In general, if the separation feature initiates on the left side 4 of the forehead region 8 or on the right side 6 in the chin region 12, a counterclockwise rotation from ray 25 to ray 29 indicates a positive angle of disposition 23. In contrast if these rotations are clockwise, the angle of disposition is negative. Similarly, in general, if the separation feature initiates on the right side 4 of the forehead region 8 or on the left side 6 in the chin region 12, a clockwise rotation from ray 25 to ray 29 indicates a positive angle of disposition 23. In contrast if these rotations are counterclockwise, the angle of disposition is negative.

In a preferred embodiment, the angle of disposition 23 of the separation feature is at least about +5°. In a further preferred embodiment the angle of disposition 23 is at least about +15°, more preferably from about at least about +20°, even more preferably at least about +30°, even more preferably at least about +40°, and even more preferably at least about 50°. to +90°. Advantageously, a separation feature having such an angle of disposition enables the laterally-extending tabs created by the separation feature to rotate about vertex V and overlap other portions of the facial mask. The angle of disposition 23 as shown in **FIG. 2** is about 70°.

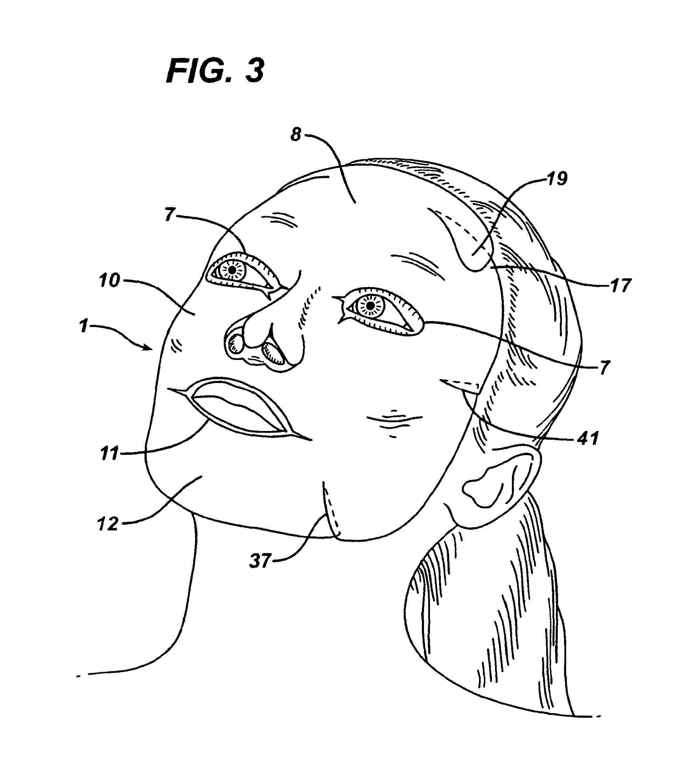

**FIG. 3** depicts the facial mask 1 of **FIGs. 1-2** being worn by a user. During use, each laterally-extending tab 19 rotates about its vertex so that the user can overlap the laterally-extending tabs 19 on other portions of the facial mask in the forehead region 8, and perhaps, if the laterally-extending tabs 19 are long enough, even on points in the mid region 10. This generally provides better, more widely variable adjustment of the facial mask dimensions, as well as better adherence to the face and a firming feeling of the facial mask against the skin.

To further increase the "substrate-to-substrate overlap," it is desirable that the separation feature, particularly a slit, have a length that is at least about 20 cm, separation feature, particularly slit, have length that is at least about 20 cm, preferably at least about 2 cm, such as from about 2 cm to about 15 cm, preferably from about 2 cm to about 10 cm, more preferably from about 3 cm to about 10 cm, and most preferably from about 3 cm to about 8 cm. The length of slit 15 is the distance from vertex V to initiation point A, the distance AV, as shown in **FIG. 2.** If the separation feature is curved, the length of the entire curved separation feature is measured.

In a particularly preferred embodiment, in order to maximize the performance of the separation feature, its angle of disposition is at least about +15° and its length is from about 2 cm to about 15 cm. In yet another particularly preferred embodiment, its angle of disposition is at least about +15° and its length is from about 2 cm to about 15 cm; more preferably its angle of disposition is at least about +20° and its length is from about 2 cm to about 10 cm; and even more preferably its angle of disposition is at least about +30° and its length is from about 3 cm to about 10 cm; and even more preferably its angle of disposition is at least about +40° and its length is from about 3 cm to about 10 cm.

Note that while in the above description the angle of disposition is calculated using the point of initiation of a slit, it is also possible to calculate the angle of disposition from any point along the separation feature.

Returning to **FIGs. 1** and **2,** the facial mask 1 may further include additional separation features. For example, facial mask 1 includes slits 37 that terminate in the chin region 12 and slits 41 that terminate in a mid portion 10. Facial mask 1 also comprises interior slits 43 that terminate at openings 7 for the eyes, and interior slits 42 that terminate at opening 11 for the mouth. Slits 37, 41 may have lengths and/or angles of disposition as described above, or their particular angle(s) of disposition may be considerably smaller. In **FIGs. 1-2,** slits 37 have angles of disposition that are essentially zero.

While **FIGs. 1-3** depict a separation feature that is a slit, one can achieve the same functional benefits with separation features having other geometries. For example, rather than a slit, the separation feature may be a notch. As used herein, a "notch" means a separation feature that generally has parallel sides and a finite width, such as may be formed by removing a rectangle of material from the water-insoluble substrate. Preferably, the notch has an aspect ratio that is from about 100:1 to about 1:1.

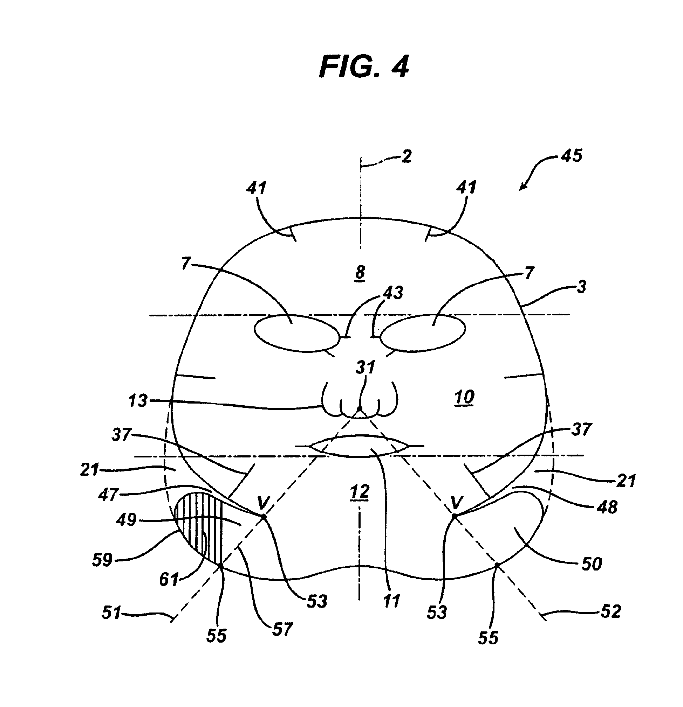

Similar functionality can be created by a separation feature that has tapering or irregular or curved boundaries that otherwise make it difficult to determine a "direction" or angle of disposition associated therewith. Applicants denote such separation features as "wedges," as illustrated in **FIG. 4** depicting another embodiment of the invention. Facial mask 45 includes many features that are more or less identical to those described with respect to **FIGs. 1-3.** Facial mask 45 includes a pair of wedges 47, 48. The wedges 47, 48 each define an area of indentation 21 that is from about 0.5 $cm^2$ to 20 $cm^2$, preferably from about 1 $cm^2$ to about 10 $cm^2$, such as from about 2 $cm^2$ to about 5 $cm^2$.

The wedges 47 are located in chin region 12. The wedges 47, 48 result in a pair of laterally-extending tabs 49, 50. Rays 51, 52 may be constructed through geometric centroid 31 and vertices V of each wedge 47, 48. Rays 51, 52 also intersect second points 55 on the perimeter 3. Preferably rays 51, 52 do not pass through any points on the perimeter other than other than vertices V and second points 55.

The boundary of laterally-extending tab 49 is defined by segment 57 (defined by ray 51 between its vertex V and its second point 55) and portion 59 of the perimeter 3. Portion 59, by nature of its curved or cup-like shape, provides a high degree of freedom of motion for laterally-extending tab 49 to rotate about vertex V and second point 55 while allowing the remainder of facial mask 45 to remain flat against the skin. Segment 57 may have a length that is at least about 1 cm, such as at least about 1.5 cm.

Specifically, laterally-extending tab 49 creates a vertical hinge having a hinge area 61 shown in cross-hatch in **FIG. 4.** Hinge area 61 comprises that portion of the laterally-extending tab 49 bounded by a line parallel to the axis of symmetry 2 and intersecting perimeter 3.

The hinge area of the laterally-extending tab is at least about 0.5 $cm^2$, preferably at least about 0.75 $cm^2$, more preferably at least about 1.5 $cm^2$, even more preferably at least about 2.0 $cm^2$. This sized hinge area enables the user to easily expand or reduce the dimensions of the facial mask.

Note that while the determination of hinge area is shown for the laterally-extending tab 49 of **FIG. 4,** hinge area can be determined in a similar manner for a slit or notch, such as the slit 15 of **FIG. 1.** In this case a ray is drawn from the geometric centroid through a point on the slit. The portion of the facial mask defined by the perimeter and a line parallel to the axis of symmetry and intersecting the perimeter comprises the hinge area.

In a particularly preferred embodiment, the facial mask comprises a slit in the forehead region having a length of about 2.5 cm and providing an angle of disposition of about 70°. A laterally-extending tab has a width of about 1.4 cm and a hinge area of about 2.4cm. The facial mask may also comprise a laterally-extending tab in the chin region having a length of 2.5 cm and a width of 2 cm (hinge area 3.6 cm)

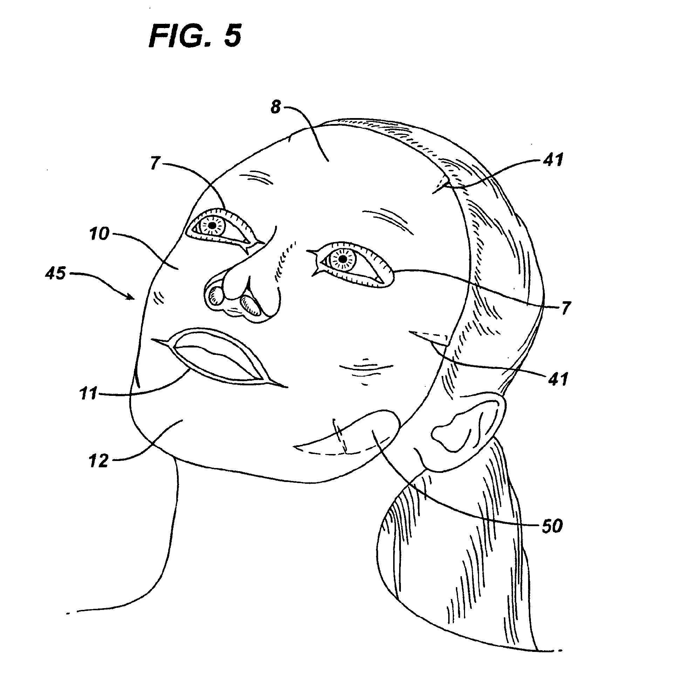

**FIG. 5** depicts the facial mask 45 worn by a user, depicting a function of laterally extending tab 50. In use, each of the laterally-extending tabs 49, 50 are able to rotate about respective vertexes 53 and second points 55 so that the user can position the laterally-extending tabs 49, 50 on top of other portions of the mask in the chin region 12, and perhaps, if the laterally-extending tabs 49, 50 are long enough, even in the mid region 10.

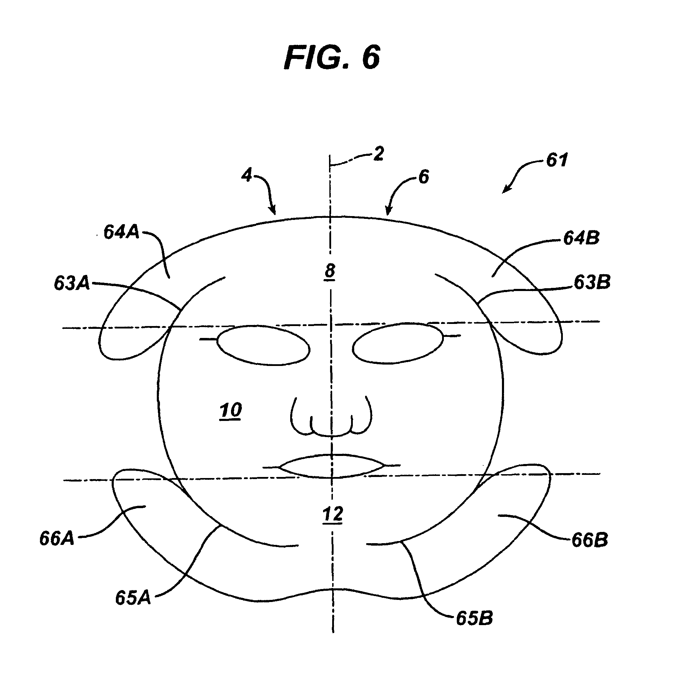

**FIG. 6** depicts a plan view of another embodiment of the invention. Facial mask 61 includes a pair of slits 63A, 63B in forehead region 8. Slits 63A, 63B result in the formation of a pair of laterally-extending tabs 64A, 64B. Facial mask 61 also includes a pair of slits 65A, 65B located in the chin region 12. Slits 65A, 65B result in the formation of a pair of laterally-extending tabs 66A, 66B.

In one embodiment, slits 63A and 65A are long enough that laterally-extending tabs 64A and 66A overlap. Similarly, slits 63B and 65B are long enough that laterally-extending tabs 64B and 66B overlap. Any of slits 63A, 63B, 65A, or 65B may have an angle of disposition similar to that described above for slit 15 of **FIGs. 1-3.** Any of slits 63A, 63B, 65A, or 65B may be replaced with notches or wedges, such as wedges 47, 48 of **FIGS. 4-5.**

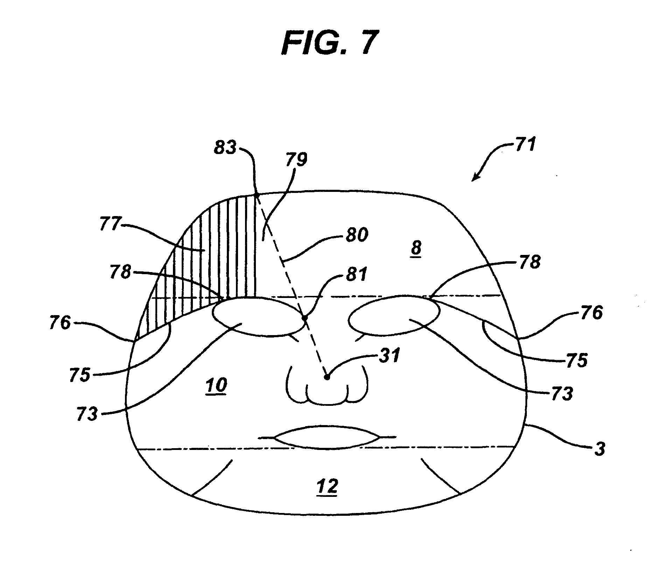

**FIG. 7** depicts yet another embodiment of the invention in which a facial mask 71 includes openings 73 for the eyes. Adjoining each opening 73 is a slit 75 that extends to the perimeter 3. The slits 75 each extend from a point 76 on the perimeter to a point 78 at the opening 73. Laterally-extending tabs 79 are bounded by (1) ray 80 that connects geometric centroid 31, point 81 on the opening 73, and point 83 on the perimeter 3; (2) the boundary of opening 73; (3) slit 75; and (4) the perimeter. Laterally-extending tabs 79 have a vertical hinge area 77, exemplified for the left side as the cross-hatched region. Unlike conventional slits used in middle areas of conventional facial masks, laterally-extending tabs 79 that result from the connection of the openings 73 and the slit 75 create a large hinge area in facial mask 71.

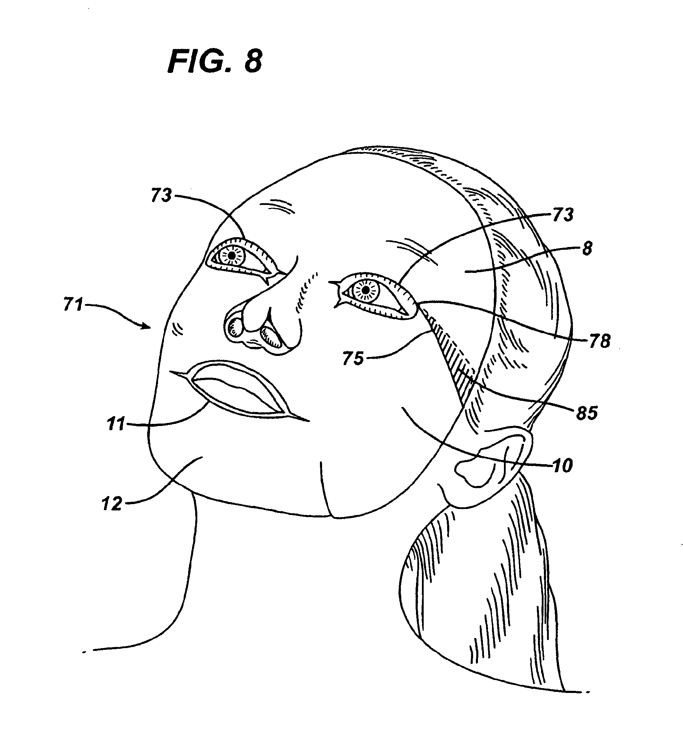

As depicted in **FIG. 8,** facial mask 71 in use is adjustable and further permits the overlap of a top section 8 with section 10 of the facial mask 71 to create an overlapping zone 85 (shown in cross-section of **FIG. 8).**

Further, in order to provide ease of use and comfort the facial mask is preferably free of straps (e.g., elastic or rubber straps) and the like that are adapted to wrap around the back of the head or back of the neck of the user.

In one embodiment of the invention, the water-insoluble substrate includes a non-woven material. As used herein, "non-woven" means that the substrate, or a layer of the substrate, is comprised of fibers that are not woven into a fabric but rather are formed into a sheet, mat, or pad layer. The fibers can either be random (i.e., randomly aligned) or they can be carded (i.e., combed to be oriented in primarily one direction). Furthermore, the non-woven substrate can be composed of a combination of layers of random and carded fibers.

In order to enhance the durability of the substrate, the non-woven material is formed such that it is not "paper" or "paper-like." As such, in this embodiment of the invention, the non-woven structure is such that more than about 50% of the fibrous mass are made of fibers having a length to diameter ratio greater than about 300. While the fibers may be staple fibers or continuous filaments, it is preferred that the fibers are staple fibers.

Non-woven substrates may be comprised of a variety of natural and/or synthetic materials. By "natural" it is meant that the materials are derived from plants, animals, insects, or byproducts of plants, animals, and insects. By "synthetic" it is meant that the materials are obtained primarily from various man-made materials or from natural materials, which have been further altered. Non-limiting examples of natural materials useful in the present invention are silk fibers, keratin fibers (such as wool fibers, camel hair fibers) and cellulosic fibers (such as wood pulp fibers, cotton fibers, hemp fibers, jute fibers, and flax fibers).

Examples of synthetic materials include, but are not limited to, those selected from the group containing acetate fibers, acrylic fibers, cellulose ester fibers, cotton fibers, modacrylic fibers, polyamide fibers, polyester fibers, polyolefin fibers, polyvinyl alcohol fibers, rayon fibers, polyurethane foam, and mixtures thereof.

Substrates made from one ore more of the natural and synthetic materials useful in the present invention can be obtained from a wide variety of commercial sources such as Freudenberg & Co. (Durham, NC USA), BBA Nonwovens (Nashville, TN USA), PGI Nonwovens (North Charleston, SC USA), Buckeye Technologies/Walkisoft (Memphis, TN USA), Sansho Shigyo K.K. (Tosa City, Kouchi, Japan), and Fort James Corporation (Deerfield, IL USA).

Methods of making non-woven substrates are also well known in the art. Such methods include, but are not limited to, air-laying, water-laying, melt-blowing, spin-bonding, or carding processes. The resulting substrate, regardless of its method of production or composition, is then generally subjected to at least one of several types of bonding operations to anchor the individual fibers together to form a self-sustaining web. The non-woven substrate can be prepared by a variety of processes including hydro-entanglement, thermally bonding, chemical bonding and combinations of these processes. Moreover, the substrates can have a single layer or multiple layers. In addition, a multi-layered substrate can include film layer(s) (e.g., aperture or non-aperture film layers) and other non-fibrous materials.

Strength or firmness of the non-woven material may be a desirable attribute. This can be achieved, for example, by the addition of binding materials, such as wet strength resins, or the material may be made of polymer binder coatings, stable fibres, e.g. based on cotton, wool, linen and the like. Examples of wet strength resins include, but are not limited to, vinyl acetate-ethylene (VAE) and ethylene-vinyl chloride (EVCL) Airflex emulsions (Air Products, Lehigh, PA), Flexbond acrylic polymers (Air Products, Lehigh, PA), Rhoplex ST-954 acrylic binder (Rohm and Haas, Philadelphia, PA), and Ethylene-vinyl acetate (EVA) emulsion (DUR-O-SET® by National Starch Chemicals, Bridgewater, NJ). The amount of binding material in the substrate may range from about 5% to about 20%, by weight, of the substrate. In one embodiment, the water-insoluble substrate is flushable, i.e., the substrate will pass through at least 10 feet of waste pipe in two toilet flushes. The material may also be biodegradable.

Non-woven materials of increased strength can also be obtained by using the so-called spunlace or hydro-entanglement technique. In this technique, the individual fibers are twisted together so that an acceptable strength or firmness is obtained without the need to use binding materials. The advantage of the latter technique is the excellent softness of the non-woven material.

In another embodiment, the water-insoluble substrate may include a hydrogel. By "hydrogel" it is meant a continuous network of polymer chains that are water-insoluble, sometimes found as a colloidal gel in which water is the dispersion medium, e.g., a multicomponent system consisting of a three-dimensional network of polymer chains and water that fills the space between macromolecules.

In one particularly preferred embodiment, the water-insoluble substrate is composed predominantly of a non-woven fabric, a hydrogel, or combinations thereof; e.g., the water-insoluble substrate may be at least about 25% by weight (exclusive of any liquid impregnate) of such materials, more preferably at least about 50% by weight.

Furthermore, the water-insoluble substrate may include thermoplastic films (e.g., polyolefin) with or without apertures. In one embodiment, the water-insoluble substrate includes a stretchable or elastic material or film that is capable of fully recovering after being placed under tension of 50% or 100% strain, such as may be included for use on the laterally-extending tabs or across the entire facial mask.

The basis weight of the water-insoluble substrate may range from about 10 grams per square meter (gsm) to about 200 gsm, such as between about 30gsm and about 100gsm. The water-insoluble substrate may have an average thickness that is less than about 5mm, such as between about 0.1mm and about 1mm.

In one embodiment of the invention, the non-woven material may include a superabsorbent polymer. For the purposes of the present invention, the term "superabsorbent polymer" refers to materials which are capable of absorbing and retaining at least about 10 times their weight in water under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and other material known to the art of absorbent article manufacture.

Additives may also be added in order to increase the softness of the substrates. Examples of such additives include, but are not limited to, polyols such as glycerol, propylene glycol and polyethylene glycol, phthalate derivatives, citric esters, surfactants such as polyoxyethylene (20) sorbitan esters, and acetylated monoglycerides.

Sensory attributes may also be incorporated to the insoluble non-woven substrates. Examples of such sensory attributes include, but are not limited to color, texture, pattern, and embossing of the substrate.

The water-insoluble substrate when laid flat, may cover an area that is from about 100 $cm^2$ to about 1000 $cm^2$, such as from about 200 $cm^2$ to about 500 $cm^2$, such as between about 200 $cm^2$ to about 360 $cm^2$.

Liquid Impregnate

The facial mask may include a liquid impregnate, such as may be used to moisten or wet the water-insoluble substrate. In one embodiment of the invention, the liquid impregnate is present in an amount sufficient to wet a user's facial skin when laid onto such skin. In another embodiment, the liquid impregnate is present in an amount sufficient to permit a first portion of the water-insoluble substrate to cohesively attach to a second portion of the water-insoluble substrate, wherein the first portion of the water-insoluble substrate is distant from the second portion of the water-insoluble substrate. Such cohesive attachment may be for a period of time of at least about 5 minutes when the water-insoluble substrate is laid on the face and the user face is in an upright, vertical orientation.

In order to provide sufficient drapeability of the facial mask, the liquid impregnate may be present in an amount that is at least about 5% by weight of the weight of the water-insoluble substrate alone. More preferably the liquid impregnate is present in an amount that is at least about 50%, even more preferably at least about 100%, even more preferably at least about 200%, such as from about 200% to about 300% of the weight of the water-insoluble substrate. By having the liquid impregnate present in this amount, the liquid impregnate may be readily transferred to skin placed in contact with the water-insoluble substrate. To further enhance the transfer of the liquid impregnate to the skin of the user, such as for a hydrating facial mask, the liquid impregnate may be present in an amount greater than about 50% by weight, such as greater than about 65%, such as between about 65% to about 95% by weight of the water-insoluble substrate.

The liquid impregnate may include an aqueous phase, an oily/hydrophobic phase, a gel phase, or a mixture of these phases. In one desirably embodiment, the liquid impregnate includes an aqueous phase, and even more preferably, the aqueous phase is an external phase (in which an oily phase or particulate phase may be dispersed, suspended or emulsified).

In one embodiment, the liquid impregnate has a viscosity that is less than about 10,000 centipoise (cps), when measured using a Brookfield digital viscometer, Model DV-II+ Version 3.2 according to the operating instructions set forth in Manual No. M/92-161-H895, such as having a viscosity less than about 5000 cps, such as less than about 1000 cps. Such low viscosity liquid impregnates tend to be aesthetically pleasing to the user.

The liquid impregnate may include solvents such as water, including those that are humectant such as glycols including glycerin or propylene glycol, or alcohols such as isopropyl alcohol or ethanol. In one preferred embodiment, the liquid impregnate includes water, such as in a concentration of at least about 40%, more preferably at least about 60%, and even more preferably at least about 80% in the liquid impregnate.

The liquid impregnate may include any of various ingredients known to the art of facial mask preparations, including: hydrophobic emollients such as fatty esters including esters of glycerin, fatty alcohols, hydrophobic polymeric emollients; sensory agents such as menthol and methyl lactate), chelating agents such as EDTA), preservatives such as parabens, and other conventional cosmetic adjuvants, such as dyes, opacifiers (e.g., titanium dioxide and zinc oxide), pigments, fragrances, and microcapsules, such aminoplast microcapsules. One particular example of suitable microcapsules are polyoxymethyene melamine urea (PMU) microcapsules, commercially available as Pontenza Dimethicone from Reed-Pacific of Dural, Austrialia. Another such example is PMU Microcapsules (32 Micron Encapsulated Mineral Oil and Jojoba Oil), available from 3M Company of St. Paul, MN.

Benefit Agents

In one embodiment of the invention, the water-insoluble substrate includes one or more benefit agents. What is meant by an "benefit agent" is a compound (e.g., a synthetic compound or a compound isolated from a natural source) that has a cosmetic ortherapeutic effect, wherein the therapeutic effects are not part of the claimed method, on the skin including, but not limited to, lightening agents, darkening agents such as self-tanning agents, anti-acne agents, shine control agents, anti-microbial agents, anti-inflammatory agents, antifungals, anti-parasite agents, external analgesics, sunscreens, photoprotectors, antioxidants, keratolytic agents, moisturizers, nutrients, vitamins, energy enhancers, anti-perspiration agents, astringents, deodorants, hair growth inhibitors, anti hair-loss agents, hair growth promoters, hair removers, skin-firming agents, anti-callous agents, anti-aging agents such as anti-wrinkle agents, skin conditioning agents, allergy inhibitors, antiseptics, external analgesics, antipruritics, antihistamines, antiinfectives, anticholinergics, vasoconstrictors, vasodilators, wound-healing promoters, peptides, polypeptides, proteins, deodorants, anti-perspirants, film-forming polymers, counterirritants, enzymes, enzyme inhibitors, poison ivy treatment agents, poison oak treatment agent, burn treatment agents; anti-diaper rash treatment agents; prickly heat agents; herbal extracts; flavenoids; sensates; anti-oxidants, keratolytics; sunscreens; and anti-edema agents; and combinations thereof.

In one embodiment of the invention, the benefit agent is selected from, but not limited to, hydroxy acids, benzoyl peroxide, sulfur resorcinol, ascorbic acid and its derivatives, D-panthenol, hydroquinone, octyl methoxycinnimate, titanium dioxide, octyl salicylate, homosalate, avobenzone, polyphenolics, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, capper salts such as copper chloride, peptides containing copper, coenzyme Q10, lipoic acid, amino acids such a proline and tyrosine, lipo amino acids such as capryloyl glycine and sarcosine, vitamins, lactobionic acid, acetyl-coenzyme A, niacin, riboflavin, thiamin, ribose, electron transporters such as NADH and FADH2, and other botanical extracts, and salt, esters, and derivatives thereof. The benefit agent will typically be present in an amount of from about 0.001 % to about 20% by weight of the liquid impregnate, e.g., about 0.01 % to about 10% such as about 0.1 % to about 5%.

Examples of vitamins include, but are not limited to, vitamin A, a vitamin B such as vitamin B3, vitamin B5, and vitamin B12, vitamin C, vitamin K, and vitamin E, and salts, esters, and derivatives thereof. (e.g., retinyl palmitate, ascorbyl acetate, and tocopherol acetate).

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbic acid glucoside, magnesium ascorbyl phosphate, and ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isofavonoids and their derivatives (e.g., genistein and diadzein), extracts containing resveratrol and the like. Examples of such natural extracts include grape seed, green tea, pine bark, and propolis.

Examples of botanical extracts include, but are not limited to legumes such as Soy, Aloe Vera, Feverfew, Hedychium, Rhubarb, Portulaca, Cedar Tree, Cinnamon, Witch Hazel, Dandelion, Chinese Angelica, Turmeric, Ginger, Burnet, Houttuynia, Coix Seed, and Thyme. What is meant by a "botanical extract" is a blend of two or more compounds isolated from a plant.

In one embodiment of the invention, the benefit agent is designed for application on the forehead region and includes, but is not limited to: oil-control agents such as titanium dioxides, alcohols, botanical extracts, and talc; pore refining agents such as alpha-hydroxy acids, beta-hydroxy acids, and enzymes; anti-acne agents such as benzoyl peroxide, salicylic acid, trichlorcarban, triclosan, azelaic acid, clindamycin, adapalene, erythromycin, sodium sulfacetamide, retinoic acid, and sulfur; oil-absorbing agents such as titanium dioxides and clays; shine control agents such as silicones, alcohols, talc, and clays; dark spot reduction agents such as vitamin C, hydroquinone, botanical extracts, alpha-hydroxy acids, beta-hydroxy acids, and retinoids; and/or wrinkle/fine-line reduction agents such as retinoids, alpha-hydroxy acids, and enzymes.

In another embodiment of the invention, the benefit agent is designed for application around the mouth and includes, but is not limited to: hydration/moisturization agents such a glycerin, silicone, glycols, botanical extracts, and esters; pore-refining agents; anti-acne agents; vasodilators such as niacinamide and horsechesnut extract; vasoconstrictors such as caffeine and botanical extracts; skin-lifting agents such as (e.g., copper containing peptides, dimethyaminoethanol, and polymers); skin-firming polymers; wrinkle/fine-line reduction agents; depigmenting/skin lightening agents such as vitamin C, hydroquinone, botanical extracts, alpha-hydroxy acids, beta-hydroxy acids, retinoids, arbutin, and kojic acid; and depilatory/hair reducing agents such as soy extracts, n-acetyl-cysteine, and isoflavones.

While various combinations are contemplated, under one non-limiting example, one or more benefit agents are selected from the group consisting of ascorbic acid and its derivatives, alpha-hydroxy-acids, beta-hydroxyacids, alkanolamines, proteins, enzymes, and enzyme activators, and combinations thereof are in the liquid impregnate, and one or more benefit agents are selected from the group consisting of retinoids, tocopherols, enzymes, enzyme activators, and combinations thereof are within the liquid core.

In one embodiment of the invention, the product comprises an enzyme such as a lignin peroxidase and a suitable activator such as a peroxide (e.g., hydrogen peroxide) as described in WO 2004/052275.

Packaging of Product

In one embodiment of the invention, the product is in finished packaged form inside a package. In one embodiment, the package is a container such as a plastic, metal or glass tube, tub, pouch or jar containing the water-insoluble substrate. The product may further contain additional packaging such as a plastic or cardboard box for storing one or more of such containers (e.g., a package of two to twenty individual products). Non-limiting examples of material that may be used to manufacture such containers include aluminum, polypropylene, polyethylene, and/or polyesters. In one embodiment of the invention, the package is substantially air-impermeable.

In one embodiment of the invention, the product includes instructions directing the user to apply the facial mask to the skin, such as to the face. In one embodiment, where the water-insoluble substrate contains a liquid impregnate that is present in an amount at least about 5% by weight of the weight of the water-insoluble substrate, the instructions direct the use to apply the product directly to the skin. In another embodiment where the water-insoluble substrate contains a liquid impregnate that is present in an amount at least about 5% by weight of the weight of the water-insoluble substrate or a product that does not contain any liquid impregnate, the instructions direct the use to apply a liquid to the facial mask prior to application to the skin (e.g. to add water, a toner, or a cleanser to the product).

In one embodiment, the instructions direct the user to apply the product for the benefit of changing the appearance of the tone and/or color of the skin.

The instructions may direct contacting the facial mask with the skin (e.g., the face) for a period of time, such as from about 10 seconds to about 1 hour (e.g., such as from about 1 minute to about 15 minutes). The user may also be directed to massage any liquid remaining on the skin after removal of the water-insoluble substrate. Such massaging may facilitate imparting improved color/tone uniformity in the skin of the subject.

Method of Making and Using the Product

Facial masks of the present invention may be made by various conventional methods, known to those skilled in the art. For example, a water-insoluble substrate, such as a sheet of non-woven material optionally perforated or cut to a pre-determined size to form a "blank," such a size and shape suitable to fit over a human face, may be formed by methods already discussed. Openings may optionally be cut out of the blank corresponding to the eyes, nose, and/or mouth. Using a "subtractive" method one or more separation features such as slits, notches, and wedges may be sliced, cut or punched out of the blank. In one embodiment, the separation feature is formed by weakening or perforating the substrate rather than by slitting or removing material. Consistent with these embodiments, the facial mask may be of an "integrated" structure, in that the entire facial mask is essentially uniform throughout its area, as viewed from the top in a plan view.

In an alternative embodiment, the facial mask may be at least partially formed by an "additive" process, *i.e.* portions of the mask are stitched, bonded, or adhered together to create the separation features. After forming the water-insoluble substrate, the substrate may then be folded and placed in a plastic pouch housing or other suitable container.

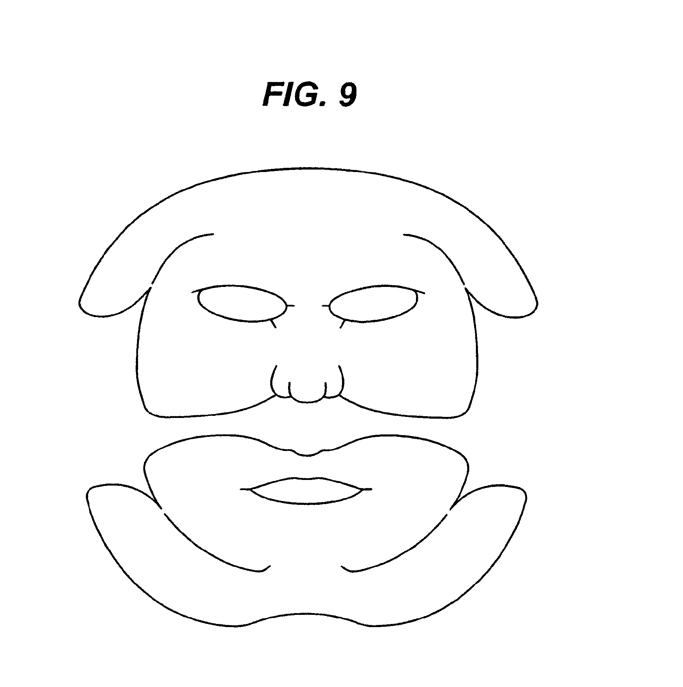

It is generally preferred that facial masks of the present invention are unitary, i.e., a one-piece mask that can be readily picked up with a user's hands and transported as single cohesive unit, and this single cohesive unit is generally adapted to cover substantially the entire face of a user. However, the facial mask may also be formed from two or more pieces, for example as shown in **FIG. 9.** Suitable two piece mask designs with slits include a top piece that includes most or all of the forehead portion and a bottom piece that includes most or all of the chin portion. For embodiments in which there are two pieces, in order to determine the geometric centroid, angle of disposition, etc., the two pieces are placed in relative positions with respect to one another that are suitable for in-use. For embodiments of the invention in which the facial mask has a slit having an angle of disposition substantially greater than 0°, the facial mask may be either a single piece mask or a multi-piece mask, such as a two piece mask.

The optional liquid impregnate may be prepared by mixing ingredients such as water and one or other ingredients and/or more benefit agents together to form a uniform liquid. The resulting liquid impregnate may then be poured into the housing. Alternatively, the impregnate may be sprayed or otherwise distributed about the substrate.

The resulting facial mask may be individually sealed in the housing or placed along with other water-insoluble substrates together into a single housing. Multiple packaged substrates may be grouped together in an outer container, such as a box.

The user may place or position the one or more water-insoluble substrates on the facial skin so as to cover substantially the entire face. The user may overlap a first portion, such as a laterally extending tab of the one or more water-insoluble substrates onto a second portion of the one or more water-insoluble substrates such that first portion of overlaps the second portion (see for example, **FIGs. 3, 5 and 8**). The overlapping may be sufficient to provide adherence between said first portion and said second portion. As such, the user may walk around or perform other actions without the facial mask dislodging.

The following example illustrates details of embodiments of the invention, without limiting it in any matter.

Example

The following is an example of a hydrating mask that includes a water-insoluble substrate for application to the face according to the invention.

A water-insoluble substrate formed from a sheet of nonwoven fibers, (KP9560, a blend of 35% rayon and 45% pulp and 10% PET, 60 grams per square meter, commercially available from Sansho Shigyo K.K. of Tosa City, Kouchi, Japan) was cut to size a shape to fit a human face. The outer dimensions were about 20.3cm X 23.2 cm. Openings were cut out of the sheet corresponding to the eyes, nose, and mouth. A facial mask having a design similar to that shown in **FIG. 1** was made. Using the same materials, others having a design similar to that described in **FIGs. 4, 6 and 7** were made.

A liquid impregnate was prepared similar to the liquid impregnate used in commercially available NEUTROGENA Fine Fairness Mask with Vitamin C, commercially available from Neutrogena Corporation, Los Angeles, California. Each of the masks above was impregnated with this impregnate. The liquid-insoluble substrate was folded and placed in a plastic pouch housing.

The facial masks were worn by one or more users. Each of the facial masks were well-received with respect to their ease of use, adjustability, and ability to adhere well to the skin.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

**Claims**

1. A facial mask(1) comprising:

a unitary, water-insoluble substrate sized and shaped to lie against and substantially coincident with an entire face of a human user, said water-insoluble substrate defined by a perimeter (3) and comprising a forehead region (8), a mid region (10), and a chin region (12), **characterised in that** said water-insoluble substrate further comprises at least one separation feature(15, 47) that forms a laterally-extending tab (49) having a hinge area (61) of at least 0.5 cm$^2$, in particular wherein the laterally-extending tab (49) has a hinge area (61) of at least about 0.75 cm$^2$.

2. The facial mask(1) of claim 1, wherein the separation feature (15, 47) is selected from the group consisting of slits (15), notches, and wedges (47).

3. The facial mask (1) of claim 1 or claim 2, wherein the separation feature (15, 47) extends into the forehead region (8) or the chin region (12).

4. The facial mask(1) of claim 1 or claim 2, wherein said separation feature (15, 47) extends from an opening (7) for an eye of a user to the perimeter (3) of the water-insoluble substrate.

5. The facial mask (1) of claim 1, wherein said at least one separation feature (15, 47) extends from said perimeter (3) into either said forehead region(8) or said chin region (12), and wherein said separation feature (15, 47) has an angle of disposition(23) greater than 0°, in particular wherein the angle of disposition (23) is greater than about 15°.

6. The facial mask (1) of any preceding claim, wherein the water-insoluble substrate comprises a pair of separation features (47, 48) reflected about an axis of symmetry (2), wherein each separation feature(47, 48) forms a laterally-extending tab (49, 50) having a hinge area (61) of at least 0.5 cm$^2$, in particular wherein the laterally-extending tabs (49, 50) have a hinge area(61) of at least about 0.75 cm$^2$.

7. The facial mask(1) of any preceding claim, wherein the water-insoluble substrate further comprises openings (7,11) for the eyes and mouth of the user.

8. The facial mask (1) of any preceding claim, further comprising a liquid impregnate absorbed on the water-insoluble substrate, wherein said liquid impregnate is present in an amount of at least about 5% by weight of the water-insoluble substrate, in particular wherein said liquid impregnate is present in a amount of at least about 50% by weight of the water-insoluble substrate.

9. The facial mask (1) of any preceding claim, wherein said water-insoluble substrate comprises a nonwoven fabric, a hydrogel, or combinations thereof.

10. The facial mask (1) of any preceding claim free of straps adapted to wrap around the back of the head or back of the neck of the user.

11. A non-therapeutic method of treating the facial skin of a human user, comprising:

   placing a water-insoluble substrate against the facial skin of said user such that said water-insoluble substrate covers substantially all of said facial skin, wherein a first portion of said water-insoluble substrate overlaps a second portion of said water-insoluble substrate, whereby said first portion and said second portion adhere to one another, **characterised in that** the water-insoluble substrate is as defined according to any one of claims 1 to 10.

**Patentansprüche**

1. Gesichtsmaske (1), umfassend:

   ein einheitliches, wasserunlösliches Substrat, das eine derartige Größe und Form aufweist, dass es auf dem gesamten Gesicht eines menschlichen Benutzers aufliegt und im Wesentlichen damit übereinstimmt, wobei das wasserunlösliche Substrat durch einen Umfang (3) definiert ist und eine Stirnregion (8), eine Mittelregion (10) und eine Kinnregion (12) umfasst, **dadurch gekennzeichnet, dass** das wasserunlösliche Substrat ferner mindestens ein Trennmerkmal (15, 47) umfasst, das eine sich längs erstreckende Lasche (49) bildet, welche einen Gelenkbereich (61) von mindestens 0,5 cm$^2$ aufweist, wobei insbesondere die sich längs erstreckende Lasche (49) einen Gelenkbereich (61) von mindestens etwa 0,75 cm$^2$ aufweist.

**2.** Gesichtsmaske (1) nach Anspruch 1, wobei das Trennmerkmal (15, 47) ausgewählt ist aus der Gruppe bestehend aus Schlitzen (15), Kerben und Keilen (47).

**3.** Gesichtsmaske (1) nach Anspruch 1 oder Anspruch 2, wobei sich das Trennmerkmal (15, 47) in die Stirnregion (8) oder die Kinnregion (12) erstreckt.

**4.** Gesichtsmaske (1) nach Anspruch 1 oder Anspruch 2, wobei sich das Trennmerkmal (15, 47) von einer Öffnung (7) für ein Auge eines Benutzers zum Umfang (3) des wasserunlöslichen Substrates erstreckt.

**5.** Gesichtsmaske (1) nach Anspruch 1, wobei sich das mindestens eine Trennmerkmal (15, 47) vom Umfang (3) entweder in die Stirnregion (8) oder die Kinnregion (12) erstreckt, und wobei das Trennmerkmal (15, 47) einen Dispositionswinkel (23) aufweist, der größer als 0 ° ist, wobei insbesondere der Despositionswinkel (23) größer als etwa 15 ° ist.

**6.** Gesichtsmaske (1) nach einem der vorhergehenden Ansprüche, wobei das wasserunlösliche Substrat ein Paar Trennmerkmale (47, 48), reflektiert um eine Symmetrieachse (2), umfasst, wobei jedes Trennmerkmal (47, 48) eine sich längs erstreckende Lasche (49, 50) bildet, die einen Gelenkbereich (61) von mindestens 0,5 cm$^2$ aufweist, wobei insbesondere die sich längs erstreckenden Laschen (49, 50) einen Gelenkbereich (61) von mindestens etwa 0,75 cm$^2$ aufweisen.

**7.** Gesichtsmaske (1) nach einem der vorhergehenden Ansprüche, wobei das wasserunlösliche Substrat ferner Öffnungen (7, 11) für Augen und Mund des Benutzers umfasst.

**8.** Gesichtsmaske (1) nach einem der vorhergehenden Ansprüche, ferner umfassend ein flüssiges Imprägnat, absorbiert auf dem wasserunlöslichen Substrat, wobei das flüssige Imprägnat in einer Menge von mindestens etwa 5 Gew.% des wasserunlöslichen Substrates vorliegt, wobei insbesondere das flüssige Imprägnat in einer Menge von mindestens etwa 50 Gew.% des wasserunlöslichen Substrates vorliegt.

**9.** Gesichtsmaske (1) nach einem der vorhergehenden Ansprüche, wobei das wasserunlösliche Substrat einen Vliesstoff, ein Hydrogel oder Kombinationen davon umfasst.

**10.** Gesichtsmaske (1) nach einem der vorhergehenden Ansprüche, frei von Bändern, die zum Wickeln um den Hinterkopf oder Nacken des Benutzers ausgelegt sind.

**11.** Nichttherapeutisches Verfahren zur Behandlung der Gesichtshaut eines menschlichen Benutzers, umfassend:

Auflegen eines wasserunlöslichen Substrates auf die Gesichtshaut des Benutzers, derart dass das wasserunlösliche Substrat im Wesentlichen die gesamte Gesichtshaut bedeckt, wobei ein erster Abschnitt des wasserunlöslichen Substrates einen zweiten Abschnitt des wasserunlöslichen Substrates überlappt, wobei der erste Abschnitt und der zweite Abschnitt aneinander haften, **dadurch gekennzeichnet, dass** das wasserunlösliche Substrat gemäß einem der Ansprüche 1 bis 10 definiert ist.

## Revendications

**1.** Masque facial (1), comprenant un substrat unitaire insoluble dans l'eau qui est dimensionné et configuré de manière à s'appuyer contre et à épouser sensiblement la totalité du visage d'un utilisateur humain, ledit substrat insoluble dans l'eau étant défini par un périmètre (3) et comprenant une région de front (8), une région intermédiaire (10) et une région de menton (12), **caractérisé en ce que** ledit substrat insoluble dans l'eau comprend en outre au moins une caractéristique de séparation (15, 47) qui forme une patte s'étendant latéralement (49) qui présente une région de charnière (61) d'au moins 0,5 cm$^2$, en particulier dans lequel la patte s'étendant latéralement (49) présente une région de charnière (61) d'au moins environ 0,75 cm$^2$.

**2.** Masque facial (1) selon la revendication 1, dans lequel la caractéristique de séparation (15, 47) est sélectionnée dans le groupe comprenant des fentes (15), des encoches et des coins (47).

**3.** Masque facial (1) selon la revendication 1 ou la revendication 2, dans lequel la caractéristique de séparation (15, 47) s'étend dans la région du front (8) ou la région du menton (12).

**4.** Masque facial (1) selon la revendication 1 ou la revendication 2, dans lequel ladite caractéristique de séparation (15, 47) s'étend à partir d'une ouverture (7) à partir d'un oeil d'un utilisateur jusqu'au périmètre (3) du substrat insoluble dans l'eau.

**5.** Masque facial (1) selon la revendication 1, dans lequel ladite au moins une caractéristique de séparation (15, 47) s'étend à partir dudit périmètre (3) soit dans ladite région de front (8), soit dans ladite région de menton (12), et dans lequel ladite caractéristique de séparation (15, 47) présente un angle de disposition (23) qui est supérieur à 0°, en particulier dans lequel l'angle de disposition (23) est supérieur à environ 15°.

**6.** Masque facial (1) selon l'une quelconque des revendications précédentes, dans lequel le substrat insoluble dans l'eau comprend une paire de caractéristiques de séparation (47, 48) qui sont réfléchies autour d'un axe de symétrie (2), dans lequel chaque caractéristique de séparation (47, 48) forme une patte s'étendant latéralement (49, 50) qui présente une région de charnière (61) d'au moins 0,5 cm$^2$, en particulier dans lequel les pattes s'étendant latéralement (49, 50) présentent une région de charnière (61) d'au moins environ 0,75 cm$^2$.

**7.** Masque facial (1) selon l'une quelconque des revendications précédentes, dans lequel le substrat insoluble dans l'eau comporte en outre des ouvertures (7, 11) pour les yeux et la bouche de l'utilisateur.

**8.** Masque facial (1) selon l'une quelconque des revendications précédentes, comprenant en outre un liquide d'imprégnation qui est absorbé sur le substrat insoluble dans l'eau, dans lequel ledit liquide d'imprégnation est présent en une quantité d'au moins environ 5 % en poids du substrat insoluble dans l'eau, en particulier dans lequel ledit liquide d'imprégnation est présent en une quantité d'au moins environ 50 % en poids du substrat insoluble dans l'eau.

**9.** Masque facial (1) selon l'une quelconque des revendications précédentes, dans lequel ledit substrat insoluble dans l'eau comprend un tissu non tissé, un hydrogel ou des combinaisons de ceux-ci.

**10.** Masque facial (1) selon l'une quelconque des revendications précédentes exempt de sangles adaptées pour s'enrouler autour de l'arrière de la tête ou de l'arrière du cou de l'utilisateur.

**11.** Procédé non thérapeutique pour traiter la peau du visage d'un utilisateur humain, comprenant la pose d'un substrat insoluble dans l'eau contre la peau du visage dudit utilisateur, de telle sorte que ledit substrat insoluble dans l'eau recouvre sensiblement la totalité de la peau du visage, dans lequel une première partie dudit substrat insoluble dans l'eau chevauche une deuxième partie dudit substrat insoluble dans l'eau, dans lequel ladite première partie et ladite deuxième partie adhèrent l'une à l'autre, **caractérisé en ce que** le substrat insoluble dans l'eau est défini selon l'une quelconque des revendications 1 à 10.

# FIG. 1

# FIG. 2

# FIG. 3

# FIG. 4

## FIG. 5

# FIG. 6

# FIG. 7

## FIG. 8

# FIG. 9

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060104931 A1
- US 20050013784 A1
- US 20040018166 A1
- EP 1357819 B1
- WO 0101952 A1
- FR 2870698 A1
- WO 2004052275 A